# EUROPEAN PATENT APPLICATION

(11) **EP 4 282 325 A1**
(43) Date of publication of application: **29.11.2023**
(21) Application number: 23173899.8
(22) Date of filing: 17.05.2023
(51) Int. Cl.: A61B 5/00, A61B 5/103, G06T 7/00, G16H 30/00, G16H 30/20, G16H 30/40, G16H 50/30

(54) **PROCESS FOR ENCODING IMAGES OF A CONDITION OF A SCALP**

(30) Priority: 23.05.2022 IT 202200010664
(71) Applicant: Sanders S.r.l., 20124 Milano (IT)
(72) Inventor: MANCINI, Biancamaria, 00162 Roma (IT)
(74) Representative: Rapisardi, Mariacristina

(57) **Abstract**

The process for encoding images of a condition of a scalp, comprises the steps of:
- extracting from said images of the scalp a piece of information indicative of a colour of the scalp, a piece of information indicative of a state of skin fragmentation of the scalp, and a piece of information indicative of a state of sebum accumulation of the scalp;
- generating an alphanumeric code for encoding comprising at least a first code field carrying a value derived from said information indicative of colour, state of skin fragmentation and state of sebum accumulation.

## Description

The present invention refers to a process for encoding images of a condition of a scalp.

### Prior art

The present invention concerns in particular the field of trichology, a study that deals with the scalp and hair, both in the physiological condition, and in the paraphysiological condition.

Thinning and baldness are the result of a long and gradual degenerative process of the follicle whose causes are multifactorial. It is now known that, among the causes of thinning, there are the hyperseborrhea, redness and desquamation which are often associated with inflammatory states. In addition, such problems of the scalp are often associated with itching and trichodynia that greatly worsen the person's quality of life, not only from an aesthetic point of view.

The analysis of the conditions of the scalp, is normally performed by:
- a physical examination of the health state of the scalp
- an in-depth study in the micro-camera with different lens:
   ∘ 20x magnification in white light
   ∘ 200x magnification in white light

The analysis described is therefore only qualitative and produces only descriptive data. For this reason, the sensitivity of the professional in carrying the data detectable by the images viewed becomes very important.

In addition, the description of an image is expressed in qualitative terms and does not lend itself to being inserted within a database or within a numerical analysis and therefore does not facilitate the collection of data and its systematicity and repeatability.

It is therefore clear that in this context the experience and the professionalism of the operator carrying out the analysis is very important and it is clear that the variability of the result is due to the sensitivity and the experience of the operator.

The task of the present invention is therefore to provide a process for the classification of a condition of the scalp of the individual's head that allows to solve the drawbacks described in the known art.

Within the scope of this technical task, an object of the present invention is to provide a process for encoding images of a condition of a scalp that allows to encode in a standardized and objective manner a condition of the scalp.

Another object of the present invention is to provide a process for encoding images of a condition of a scalp in a systematic and repeatable manner.

Another object of the present invention is to provide a process for encoding images of a condition of a scalp which provides reliable data representative of the condition of the scalp.

These and other objects of the present invention are achieved by a process for encoding images of a condition of a scalp, characterized in that it comprises the steps of:
- extracting from the images of the scalp a piece of information indicative of a colour of the scalp, a piece of information indicative of a state of skin fragmentation of the scalp, and a piece of information indicative of a state of sebum accumulation of the scalp;
- generating an alphanumeric code for encoding comprising at least a first code field carrying a value derived from said information indicative of colour, state of skin fragmentation and state of sebum accumulation.

Preferably said alphanumeric code comprises a second code field carrying a value derived from a symptomatology including itching and/or trichodynia.

Advantageously said alphanumeric code comprises a second code field carrying a value derived from a symptomatology associated with said condition including itching and/or trichodynia.

Furthermore, the process envisages extracting from said images a piece of information indicative of the state of extension of the condition, and attributing to said alphanumeric code a third code field carrying a value derived from said piece of information indicative of the state of extension of the condition.

Other salient aspects of the invention are set forth in the following dependent claims.

Further characteristics and advantages of the invention will become more apparent from the description of a preferred but not exclusive embodiment of the process for encoding images of a scalp condition according to the present invention illustrated by way of non-limiting indication in the accompanying drawings, in which:

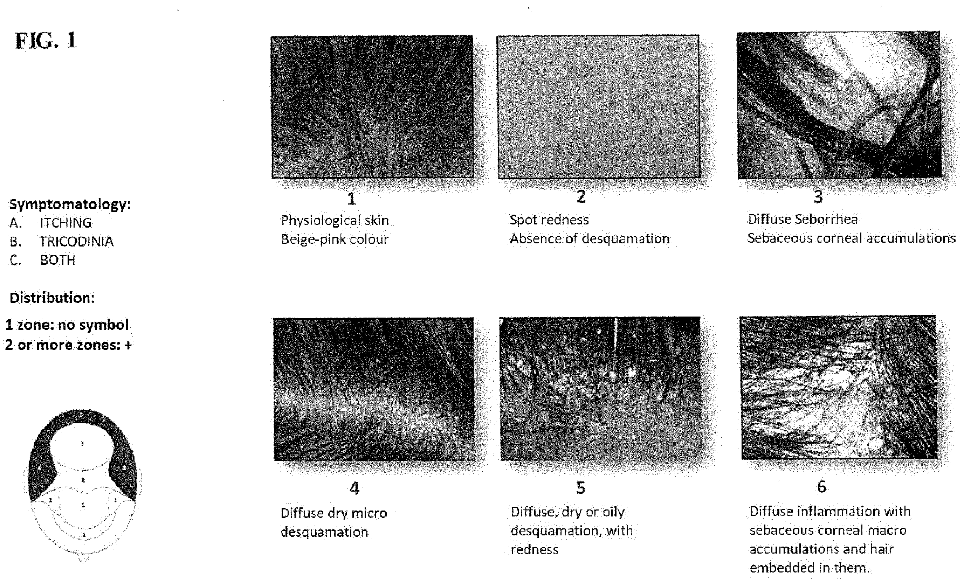

- figure 1 shows the reference scale realized through the process for the present invention;

With reference to the cited figure, a process for encoding images of a condition of a scalp of an individual's head is shown.

The process according to the present invention comprises steps of extracting from the images of the scalp a piece of information indicative of a colour of the scalp, a piece of information indicative of a state of skin fragmentation of the scalp, and a piece of information indicative of a state of sebum accumulation of the scalp.

The images represent the scalp illuminated with white light.

The images represent the scalp with different magnification factors.

The extraction of the aforesaid parameters can be performed by a human operator or automatically by analysing the images through an image analysis algorithm possibly trained by a machine learning process or even a deep learning process that involves advanced machine learning techniques based on **artificial neural networks.**

The process further envisages the step of generating an alphanumeric code for encoding comprising at least a first code field carrying a value derived from said information indicative of colour, state of skin fragmentation and state of sebum accumulation.

The alphanumeric code further comprises a second code field carrying a value derived from a symptomatology associated with said condition including itching and/or trichodynia.

The process also envisages extracting from said images piece of information indicative of the state of extension of the condition to the various anatomical districts of the scalp.

The alphanumeric code therefore also comprises a third code field carrying a value derived from said piece of information indicative of the state of extension of the condition.

The values of the code fields can also be calculated automatically using appropriate calculation algorithms.

An empty field in the code can be used to identify the minimum value attributable to the code field.

According to the invention, trichological analysis means including a dermatological lamp provided with a magnifying lens and a LED light, and a micro camera with different magnification factors are provided.

In particular, the first field has increasing numerical values that increase in proportion to the negativity of the phenomenon.

With reference to the second code field the presence of itching is identified with value defined by letter "A", the presence of trichodynia with value defined by letter "B" and the presence of both with value defined by letter "C"

With reference to the third code field the value defined by the algebraic symbol "+" identifies the simultaneous involvement of multiple zones of the head.

A preferred embodiment of the process for encoding images of a condition of a scalp will be described in detail below.

### Materials and Methods

The trichological analysis means used comprises:
- DERMATOLOGICAL LAMP: Lamp with magnifying glass and LED light, suitable for observation of small areas and magnifications in general. The magnifying glass lens is biconvex in optical glass 3 diopters, 120 mm in diameter, which allows to make observations of the scalp during the physical examination.
- MICROCAMERA: The images of the scalp and the hair shaft are obtained using the MicroCamera of the company APR Instruments and related software. The magnifications used are the panoramic, the 20x, and 200x, and possibly but not necessarily also 50FL magnifications.
   ∘ Using high-resolution panoramic optics, a photograph of an individual's head that identifies the physical examination of the general situation of the head of a specific individual can be made.
   ∘ A photograph of the state of the scalp is obtained through a 200x magnification.
   ∘ A photograph of the state of the scalp and of the shafts is obtained through a 20x magnification.
   ∘ A photograph of the state of the scalp with particular reference to the accumulation of sebum is obtained through a 50 FL magnification. The 50 FL optic is a scalp analysis tool in support of 200x that highlights the accumulation of sebum through the oxidation of sebum. Sebum accumulations appear with a fluorescence of different intensity depending on their degree of oxidation.

### ENCODING OF THE IMAGES

The images of the scalp are acquired with different scalp illuminations and different magnification factors.

For example, the images are acquired with various lens types: panoramic, 200x, 20x and 50 FL.

In particular, 6 macro types of recurring conditions were identified. Each of the 6 macro-types can be divided into sub-categories depending on the associated symptomatology and the extent of the problem.

### CLASSIFICATION PARAMETERS

The parameters used for encoding images are precise and recognizable parameters and are divided into: primary physical parameters, secondary parameters and tertiary parameters.

The primary physical parameters identify the type of condition and comprise: colour (particularly redness), skin integrity (presence or absence of desquamation) and oiliness (seborrhea).

The secondary parameters identify a symptomatology associated with the skin condition. In particular the associated symptomatology comprises itching and trichodynia.

The tertiary parameters instead identify a localization zone of the skin condition on the individual's head. The anatomical reference zones are: frontal zone, median or mid scalp zone, vertex (zone where the frontal bone meets the parietal bone), parietal and occipital.

It is defined then a scale of codes where: the possible values of the first code field vary depending on the number and type of primary physical parameters altered; the possible values of the second code field vary depending on the secondary parameters; and the possible values of the third code field vary depending on the tertiary parameters.

The values of the first field are progressive numbers , the values of the second field are letters and the values of the third field are algebraic signs.

A value of the first field can be associated with a value of the second field and/or a value of the third field.

The values in the second field include the letter "A" (itching only), "B" (trichodynia only) or "C" (simultaneous presence of itching and trichodynia).

In the case of an associated symptomatology, it will therefore be associated with the numerical value of the first code field a literal value of the second code field chosen from the letter "A" (itching only) "B" (trichodynia only) or "C" (simultaneous presence of itching and trichodynia). The same will be done for the tertiary parameters identifying the distribution of the skin condition on the individual's head. It will then be associated with the numeric value of the first code field an algebraic value of the third code field indicated with a "+" when the zones of the head affected by the condition are simultaneously more than one.

Below are reported by way of example 6 values of the first code field.

The numeric order reflects the increasing degree of importance of the condition, that is, it starts from the value 1 that identifies a situation of normality, that is, physiological skin, to reach the value 6 that represents a very compromised skin.

### FIRST VALUE FIELD CODE 1

As shown in Figure 1, the value 1 represents images of physiological skin, which has a pinkish beige colouration, in the absence of oiliness, redness and desquamation.

### Primary physical parameters associated with value 1 of the first code field

i. Colour (redness) NO
ii. Skin integrity (desquamation) YES
iii. Oiliness (seborrhea) NO

In the case of value 1 of the first code field, since they are images of physiological skin, there are no symptomatologies associated with the skin conditions.

The same applies to the tertiary parameters, which are not taken into account in the case of images of the skin in physiological condition.

The table below (Table 1) summarizes the primary, secondary and tertiary parameters of the value 1 of the first code field.

**Table 1**

| **PARAMETERS** | **PHYSICAL EXAMINATION** |
|---|---|
| Colour (presence of redness) | NO |
| Skin integrity (absence of desquamation) | YES |
| Oiliness (presence of seborrhea) | NO |
| Associated symptomatology | NO |
| Localization zone of the skin condition | NO |

Example: images of a physiological skin will be classified with first field of the code of value 1. If the person also has itching, the images will be classified with second field of the code of value A, with the resulting code 1A.

### FIRST VALUE FIELD CODE 2

As shown in Figure 1, the value of the first code field 2 represents images of skin presenting diffuse or spot redness, in the absence of physiological desquamation and oiliness.

### Primary physical parameters associated with the value of the first code field 2:

**i. Colour (redness) YES**
ii. Skin integrity (desquamation) YES
iii. Oiliness (seborrhea) NO

### Secondary parameters:

### Associated symptomatology (itching and trichodynia): YES or NO

In case of symptomatology, the value of the second code field "A", "B" or "C" must be specified:
A. ITCHING
B. TRICHODYNIA
C. BOTH SYMPTOMATOLOGIES

**Tertiary parameters:** the zone affected by the condition (zonal-diffuse). Addition will be made in the third field code of a value "+" when the zones of the head affected by the skin condition are more than one at the same time.

Example: images of reddened skin superiorly (frontal, mid scalp and vertex) with trichodynia will be classified with 2B+.

### FIRST VALUE FIELD CODE 3

As shown in Figure 1, the value of the first code field 3 represents images of skin presenting a dry micro desquamation that is indicative of dehydration or of an altered microbial component that no longer allows the maintenance of the correct hydrolipidic film and the integrity of the epidermis. Often associated with an altered cell turnover.

### Primary physical parameters associated with the value of the first code field 3:

i. Colour (redness) NO
**ii. Skin integrity (desquamation) NO**
iii. Oiliness (seborrhea) NO

### Secondary parameters:

### Associated symptomatology (itching and trichodynia): YES or NO

In case of symptomatology, the value of the second code field "A", "B" or "C" must be specified:
A. ITCHING
B. TRICHODYNIA
C. BOTH SYMPTOMATOLOGIES

**Tertiary parameters:** the zone affected by the condition (zonal-diffuse). Addition will be made of a value of the third code field "+" when the zones of the head affected by the skin condition are more than one at the same time.

Example: images of dry desquamation, without symptomatology, all over the scalp will be classified with code 3+.

### FIRST VALUE FIELD CODE 4

As shown in Figure 1, the value of the first code field 4 represents images of skin presenting diffuse seborrhea with sebaceous corneal accumulations. Seborrhea is present together with macro-corneal accumulations to which it acts as a binder, present especially close to the follicular ostium and as sleeves along the first section of the outgoing shaft.

### Primary physical parameters of the value of the first code field 4:

i. Colour (redness) NO
**ii. Skin integrity (desquamation) NO**
**iii. Oiliness (seborrhea) YES**

### Secondary parameters:

### Associated symptomatology (itching and trichodynia): YES or NO

In case of symptomatology, the value of the second code field "A", "B" or "C" must be specified:
A. ITCHING
B. TRICHODYNIA
C. BOTH SYMPTOMATOLOGIES

**Tertiary parameters:** the zone affected by the condition (zonal-diffuse). Addition will be made of a value of the third code field "+" when the zones of the head affected by the skin condition are more than one at the same time.

Example: images of sebaceous corneal accumulations on the frontal, with itching and trichodynia as symptomatology, will be classified with code 4C.

### FIRST VALUE FIELD CODE 5

As shown in Figure 1, the first field value code 5 represents images of skin presenting diffuse, dry or oily desquamation, with redness.

Seborrhea is present together with macrocorneal accumulations to which it acts as a binder, complicating the picture is a diffuse redness index of the associated inflammation.

The first field code value 5 associates the conditions of the first field code values 2, 3 and 4.

### Primary physical parameters of the first field value code 5:

**i. Colour (redness) YES**
**ii. Skin integrity (desquamation) NO**
**iii. Oiliness (seborrhea) YES**

### Secondary parameters:

### Associated symptomatology (itching and trichodynia): YES or NO

In case of symptomatology, the value of the second code field "A", "B" or "C" must be specified:
A. ITCHING
B. TRICHODYNIA
C. BOTH SYMPTOMATOLOGIES

**Tertiary parameters:** the zone affected by the condition (zonal-diffuse). Addition will be made of a value of the third code field "+" when the zones of the head affected by the skin condition are more than one at the same time.

Example: images of sebaceous corneal accumulations on the frontal and parietal, redness with itching as symptomatology, will be classified with code 5A+.

### FIRST VALUE FIELD CODE 6

As shown in Figure 1, the first field code value 6 represents images of skin presenting diffuse inflammation with sebaceous corneal plaques and/or macro accumulations

Seborrhea is present together with macrocorneal accumulations to which it acts as a binder. The formation of the accumulations is invasive and associative such that plaques or large solid accumulations that incorporate several hairs inside them are formed. Always associated with redness. Often associated with symptomatology.

The value 6 represents an exasperation of the value 5 for sebaceous corneal formations, inflammation and depth of damage.

### Primary physical parameters of code field value 6:

**i. Colour (redness) YES**
**ii. Skin integrity (desquamation) NO**
**iii. Oiliness (seborrhea) YES**

### Secondary parameters:

### Associated symptomatology (itching and trichodynia): YES or NO

In case of symptomatology, the value of the second code field "A", "B" or "C" must be specified:
A. ITCHING
B. TRICHODYNIA
C. BOTH SYMPTOMATOLOGIES

**Tertiary parameters:** the zone affected by the condition (zonal-diffuse). Addition will be made of a value of the third code field "+" when the zones of the head affected by the skin condition are more than one at the same time.

Example: images of sebaceous corneal plaques on the frontal, parietal and occipital, with itching and trichodynia as symptomatology, will be classified with code 6C+.

In practice, each value of the first code field (1-6) of the scale for the classification of a skin condition can be associated with a value of the second code relative to the associated symptomatology (A, B and C) and to a value of the third code field relative to the spread of the condition at head level ("+" when several head zones are affected simultaneously or no symbol if the affected zone is only one).

The scale for the classification of the skin condition realized by the process of the present invention allows to standardize the analytical process and to build reusable databases.

The process according to the present invention has made it possible to transform the purely qualitative data deriving from the images of the scalp to date used in the trichological analysis of the skin conditions into quantitative data thus allowing the creation of a reference scale of the skin conditions of the scalp.

The process for encoding images of a scalp condition of the individual's head thus conceived, is susceptible to numerous modifications and variants, all falling within the scope of the inventive concept.

## Claims

1. A process for encoding images of a condition of a scalp, **characterized in that** it comprises the steps of:
- extracting from said images of the scalp a piece of information indicative of a colour of the scalp, a piece of information indicative of a state of skin fragmentation of the scalp, and a piece of information indicative of a state of sebum accumulation of the scalp;
- generating an alphanumeric code for encoding comprising at least a first code field carrying a value derived from said information indicative of colour, state of skin fragmentation and state of sebum accumulation.

2. The process according to claim 1, **characterized in that** said alphanumeric code comprises a second code field carrying a value derived from a symptomatology associated with said condition including itching and/or trichodynia.

3. The process according to the preceding claim, **characterized by** extracting from said images a piece of information indicative of the state of extension of the condition, and in that said alphanumeric code comprises a third code field carrying a value derived from said piece of information indicative of the state of extension of the condition.

4. The process according to any preceding claim, **characterized in that** said images are acquired by means of trichological analysis including a dermatological lamp provided with a magnifying lens and a LED light, and a micro camera with different magnification factors.

5. The process according to any preceding claim, **characterized in that** said first code field has values formed by numbers.

6. The process according to any one of claims 2 to 5, **characterized in that** said second code field has values formed by letters of the alphabet.

7. The process according to any one of claims 3 to 6, **characterized in that** said third code field has values formed by algebraic symbols.

8. The process according to any preceding claim, **characterized in that** said images represent the scalp illuminated with white light.

9. The process according to any preceding claim, **characterized in that** said images represent the scalp with different magnification factors.

10. A scalp state encoding scale obtained by a process for encoding images of the scalp according to any preceding claim.
